# EUROPEAN PATENT SPECIFICATION

(11) **EP 4 185 213 B1**
(45) Date of publication and mention of the grant of the patent: **08.10.2025**
(21) Application number: 21847000.3
(22) Date of filing: 21.07.2021
(51) Int. Cl.: A61B 10/00, A61B 10/02, C12Q 1/68, C12Q 1/6806

(54) **DESIGN OF 3D-PRINTED NASOPHARYNGEAL SWABS**
ENTWURF VON 3D-GEDRUCKTEN NASOPHARYNGEALEN TUPFERN
CONCEPTION D'ÉCOUVILLONS NASOPHARYNGIENS RÉALISÉS PAR IMPRESSION 3D

(30) Priority: 21.07.2020 US 202063054555 P
(43) Date of publication of application: 31.05.2023
(73) Proprietor: Baylor College Of Medicine, Houston, TX 77030 (US); Texas Children's Hospital, Houston, TX 77030 (US)
(72) Inventor: STAROSOLSKI, Zbigniew, Houston, TX 77030 (US); ADMANE, Prasad, Houston, TX 77030 (US); DUNN, James, Houston, TX 77030 (US); KAZINY, Brent, Houston, TX 77030 (US); ANNAPRAGADA, Ananth, Houston, TX 77030 (US)
(74) Representative: HGF
(86) International application number: PCT/US2021/042498
(87) International publication number: WO 2022/020425

(56) References cited:
- WO-A1-2004/112538
- WO-A1-2011/116481
- US-A- 5 084 005
- US-A- 5 084 005
- US-A- 5 535 756
- US-A1- 2010 069 791
- US-A1- 2013 059 748
- US-A1- 2013 116 596
- US-A1- 2013 276 815
- US-A1- 2017 065 261
- US-A1- 2017 065 261

## Description

### RELATED APPLICATIONS

This application claims the benefit of U.S. Provisional Patent Application No. 63/054,555 filed on July 21, 2020.

### FIELD OF THE INVENTION

This invention relates to Nasopharyngeal Swabs. More particularly, to swabs for children.

### BACKGROUND OF INVENTION

This work was partially funded by a grant from the Soicher Family Foundation.

Coronavirus disease 2019 (COVID-19) is a worldwide pandemic and has resulted in shortages of medical supplies, including nasopharyngeal swabs used in diagnostic tests. 3D printing provides a novel solution, and designs for 3D-printed nasopharyngeal swabs were freely distributed via a GitHub repository (https://github.com/ramaout/Covidswab). These efforts, however, had focused on swabs for use in adult patients. These adult swabs are too inflexible and too large for safe pediatric use, particularly in children younger than 3 years of age. Prior patent document US20170065261A1 describes specimen collecting devices for the collection of biological samples, including a swab as defined in the preamble of claim 1.

As such, a 3D-printed pediatric swab that replicated the dimensions of a commercial pediatric swab (COPAN Flock Technologies; Puritan Diagnostics) was evaluated by one of our pediatric emergency physicians. However, the replicated 3D-printed pediatric swab design was not sufficiently flexible and would risk damaging the nasal passages of the child. Further, simply reducing the diameter of the flexible shaft was deemed inadvisable because the use of the swab involves twisting in both directions after insertion and a thinner shaft would risk breakage in situ. Therefore, a design for novel 3D-printed swabs for use in infants and young children is contemplated herein.

### SUMMARY OF INVENTION

The invention is defined in the appended claims. In one embodiment, an improved nasopharyngeal swab is provided. The swab comprises a tip section that is elliptic-cylindrically shaped, wherein the tip section provides an arrangement of hemispherical nubs about a peripheral surface of the tip section; a shaft section below the tip section that is elliptic-cylindrically shaped, wherein shaft section is flexible for pediatric use. The swab also provides a handle section below the shaft section, wherein the handle section has larger cross-sectional dimensions that the shaft section; and a transition section that transitions the shaft section to the handle section.

**In** another embodiment, a method for evaluating nasopharyngeal swab designs is provided. The method comprises utilizing a CT scan image to create a file suitable for 3D printing, wherein further the CT scan image is from a patient 3 years old or younger; and printing a model nasopharyngeal passage with a suitable material. Additionally, the method may also involve manufacturing or 3D printing swab designs to be tested; and evaluating the swab designs with the model nasopharyngeal passage based on resistance, navigation time, or combinations thereof. The resistance is a multi-level resistance score rating ease of insertion from external nares to a posterior nasopharynx of the model nasopharyngeal passage. The navigation time is a time need to navigate from the external nares to the posterior nasopharynx.

The foregoing has outlined rather broadly various features of the present disclosure in order that the detailed description that follows may be better understood. Additional features and advantages of the disclosure will be described hereinafter.

### BRIEF DESCRIPTION OF THE DRAWINGS

For a more complete understanding of the present disclosure, and the advantages thereof, reference is now made to the following descriptions to be taken in conjunction with the accompanying drawings describing specific embodiments of the disclosure, wherein:

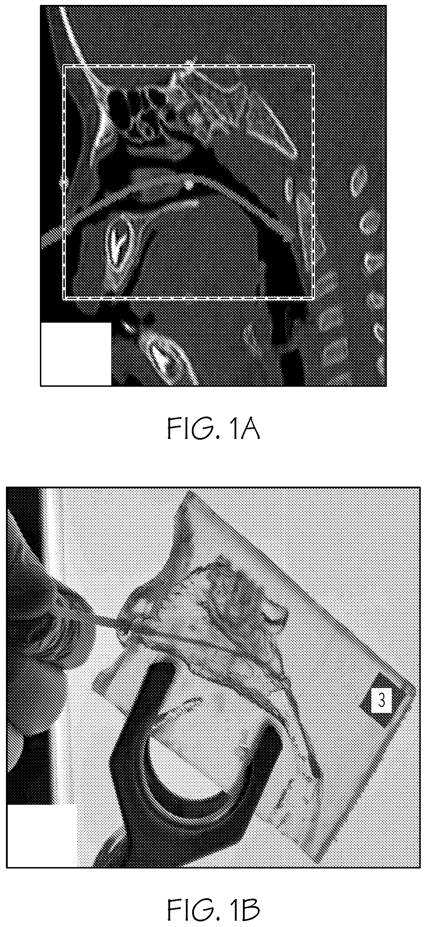

FIGS. 1A-1C-6 are illustrative embodiments of A) a sagittal slice of a maxillofacial CT scan of a 22 month old patient; B) commercial prior art flocked pediatric swab reaching the posterior nasopharynx within the 3D printed nasopharyngeal passage; and C-1 to C-6) design of the elliptical section 3D printed pediatric swab (type-E).

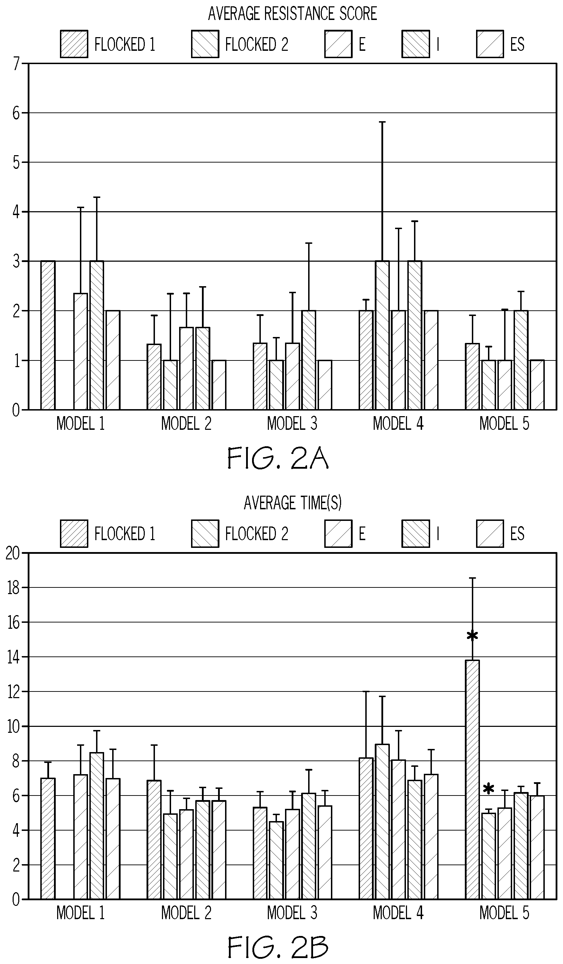

FIGS. 2A-2C show results comparison based on three repetitions and five anatomical models, including A) average performance score (the lower value the performance is better); B) Average time of procedure in passages of anatomical models (*- indicated the significant difference calculated by multiple ANOVA statistical test. Error bars represents standard dev.); and C) graph showing relation of time and performance tests results for each of tested swabs (Location closer to the graph origin indicate faster and smoother test performance).

### DETAILED DESCRIPTION

Refer now to the drawings wherein depicted elements are not necessarily shown to scale and wherein like or similar elements are designated by the same reference numeral through the several views.

Referring to the drawings in general, it will be understood that the illustrations are for the purpose of describing particular implementations of the disclosure and are not intended to be limiting thereto. While most of the terms used herein will be recognizable to those of ordinary skill in the art, it should be understood that when not explicitly defined, terms should be interpreted as adopting a meaning presently accepted by those of ordinary skill in the art.

It is to be understood that both the foregoing general description and the following detailed description are exemplary and explanatory only, and are not restrictive of the invention, as claimed. In this application, the use of the singular includes the plural, the word "a" or "an" means "at least one", and the use of "or" means "and/or", unless specifically stated otherwise. Furthermore, the use of the term "including", as well as other forms, such as "includes" and "included", is not limiting. Also, terms such as "element" or "component" encompass both elements or components comprising one unit and elements or components that comprise more than one unit unless specifically stated otherwise.

The worldwide COVID-19 pandemic has demonstrated need for improved nasopharyngeal swabs. Shortages of medical supplies include such swabs utilized for molecular diagnostic testing or the like, including COVID-19 testing. Some Radiology departments have adopted 3D printers recently to provide prints for surgical planning, patient education, or the like, and are thus uniquely positioned to produce 3D-printed nasopharyngeal swabs when needed, such as during the COVID-19 pandemic or the like. Additionally, such departments have unique access to volumetric imaging data to optimize and test swab designs. The methods for developing/evaluating swab designs and improved swabs described herein demonstrate an effective collaboration between members of clinical and research departments in accelerating development and enabling creative patient-centric solutions.

Pediatric nasopharyngeal swabs (mini-swabs) are a thinner, smaller, more flexible version of those used for adults, and are in even shorter supply during the COVID-19 pandemic than the adult swabs. Additionally, 3D-printed swabs are fundamentally different from existing commercial swab designs in the form of the swab tip: while commercial, prior swabs have flocked fibrous tips, 3D-printed swabs have solid tips with hemispheric nubs. However, both in vitro and in vivo testing have shown them to be practically identical in sample transfer for viral assay by RT-PCR both in our own labs and in other labs, thereby indicating they are suitable (Corman VM, Landt O, Kaiser M, et al. Detection of 2019 novel corona- virus (2019-nCoV) by real-time RT-PCR. Euro Surveill 2020;25:2000045 https://www.ncbi.nlm.nih.gov/pubmed/31992387; and Mullis KB, Faloona FA. Specific synthesis of DNA in vitro via a polymerase-catalyzed chain reaction. Methods Enzymol 1987;155:335-50 https://www.ncbi.nlm.nih.gov/pubmed/3431465). Of note, at the time of filing, ~45000 pediatric swabs printed using Design ES have now been used for >~30,000 patients with no reported problems.

FIGS. 1A-1C-6 show illustrative embodiments of the methods for developing/evaluating swab designs and improved swabs suitable for children. FIG. 1A shows an example of maxillofacial CT scan, which is preferably of patients 3 years old or younger, that may be utilized to provide testing model(s). CT scan image(s) may be utilized to create files suitable for 3D printing. Nasopharyngeal passages may be printed utilizing the files, such as with any suitable material, resin, or the like. FIG. 1B shows a nonlimiting example of a 3D printed nasopharyngeal passage, and further details regarding additional embodiments are discussed further below.

FIGS. 1C-1 to 1C-6 show an exemplary design for an improved swab design. The swabs are pediatric or suitable for children and infants. Additionally, the swabs suitable for 3D printing, as well as for conventional molding, injection molding and reaction injection molding methods. More particularly, the swabs may be made without the need for any additional manufacturing, such as adhesive application, flocking application, or other fiber application to the tip of the swab. The swab provides tip (i), shaft (j), shaft-to-handle transition (k), and handle (1) sections. The above sections may be a variety of lengths. The various sections of the swab are generally cylindrically-shaped, and in some embodiments in a generally elliptic-cylindrical shape. It should be noted the elliptical cross section of the shaft and/or other sections of the swab provides additional flexibility in at least one dimension, thereby providing for ease of insertion into tortuous nasopharyngeal cavities. The tip may be elliptic-cylindrically shaped and may comprise cross-sectional dimensions corresponding to a semi-major axis (1/2 of dimension a) and a semi-minor axis (1/2 of dimension d). In some embodiments, the semi-major axis, semi-minor axis, or diameter of the tip is equal to or less than 2.7 mm. In some embodiments, the semi-minor axis of the tip for elliptically-shaped embodiments is equal to or less than 1.9 mm. In some embodiments, the tip may be circular or nearly circular-shaped, where dimensions a, d are equal or nearly equal and may also be characterized as the diameter of the tip (e.g. Design I). The tip of the swab may be solid and/or unflocked (or lacking in fibers secured to the tip with an adhesive coating). The tip may also provide an arrangement of hemispherical nubs about the peripheral surface or circumference of the tip, which may extend from the top of the tip to the end of the nubbed section or to a length (b). In some embodiments, the nubbed section of the tip section is 8.5 mm in length. The arrangement of hemispherical nubs avoids the need for flocking or associated manufacturing steps needed for flocking. The tip may also optionally provide a transition tip section (c) where the size of the tip reduces to the desired size of the shaft below the tip. In some embodiments, the transition tip section has a length of 1.8 mm or less. Shaft section (j) may be elliptic-cylindrically shape and may have elliptical or circular dimensions h and g (e.g. semi-major axis & semi-minor axis or diameters). The shaft (j) is smaller, thinner, or has lower cross-sectional dimensions than the handle section (1). This allows the shaft (j) to provide the desired flexibility for swabs suitable pediatric use (e.g. FIGS. 1A-1B). In some embodiments, the semi-major axis, semi-minor axis, or diameter of the shaft section is equal to or less than 1.2 mm. In some embodiments, the semi-minor axis of the shaft section for elliptically-shaped embodiments is equal to or less than 0.8 mm. In some embodiments, the length of the shaft section is 51 mm or less. The shaft-to-handle transition section (k) is where the shaft transition to the handle or increase in cross-sectional size/dimensions to the handle section (1) below the shaft. In some embodiments, the transition section (k) has a length of 14.3 mm. The handle section (1) below the shaft section (j) and transition section (k) has larger cross-sectional dimensions than the shaft. The handle may be elliptic-cylindrically shaped and may have elliptical or circular dimensions f and e (e.g. semi-major axis & semi-minor axis or diameters). In some embodiments, the handle section has a length of 78.4 mm.

All embodiments discussed, including the dimensions and ranges, are approximations merely setting forth preferred embodiments. Variance from such dimensions is acceptable, e.g. 10% variance. Table I provides a full breakdown of three illustrative embodiments of the swabs discussed herein. Design ES showed the best results in testing - however, all three designs are deemed satisfactory.

**TABLE I: Tabularized dimensions for Improved Swab designs**

| Swab | a | b | c | d | e | f | g | h | i | j | k | l |
|---|---|---|---|---|---|---|---|---|---|---|---|---|
| Design E | 2.7 | 8.5 | 0 | 1.9 | 3.5 | 5.0 | 0.8 | 1.2 | 8.5 | 50.8 | 14.3 | 78.4 |
| Design I | 2.7 | 8.5 | 0 | 2.6 | 5.0 | 5.0 | 1.2 | 1.2 | 8.5 | 50.8 | 14.3 | 78.4 |
| Design ES | 2.7 | 8.5 | 1.8 | 1.9 | 3.5 | 5.0 | 0.8 | 1.2 | 10.4 | 49 | 14.3 | 78.4 |

| | | | | | | | | | | | | |
|---|---|---|---|---|---|---|---|---|---|---|---|---|
| Dimensions are in millimeters. Columns represents dimensions as shown in FIGS. 1C-1 to 1C-6. | | | | | | | | | | | | |

Returning to the methods for developing/evaluating swab designs, swab designs to be tested, such as the three examples above, may be manufactured or 3D printed. The prior 3D printed nasopharyngeal passage(s) may be utilized for development or evaluation of the swabs. In a nonlimiting example, at least five 3D printed nasopharyngeal passages may be utilized as anatomical models. The swabs may be evaluated based on results relating to resistance or ease of insertion, navigation time, or combinations thereof. In some embodiments, multiple test of each swab design may be performed for each anatomical model for resistance and/or navigation time. For example, swab designs may be evaluated for resistance for the anatomical models based on multi-level resistance scores and averaged (e.g. 1 - easy insertion/no resistance, 2 - medium/mild resistance, and 3 - hard/ requiring extra force). Navigation time for the anatomical models may measure time needed to navigate from external nares to the posterior nasopharynx for each swab design and may also be averaged. The design with the lowest resistance and/or navigation time may be selected as the preferred design. Average test duration and performance/resistance scores for the three designs discussed previous indicated all the designs are comparable to commercial swabs. The ES design's results indicated the design is preferable, but all designs performed satisfactory.

### Experimental Example

The following examples are included to demonstrate particular aspects of the present disclosure. It should be appreciated by those of ordinary skill in the art that the methods described in the examples that follow merely represent illustrative embodiments of the disclosure. Those of ordinary skill in the art should, in light of the present disclosure, appreciate that many changes can be made in the specific embodiments described and still obtain a like or similar result without departing from the scope of the present disclosure.

### MATERIALS AND METHODS

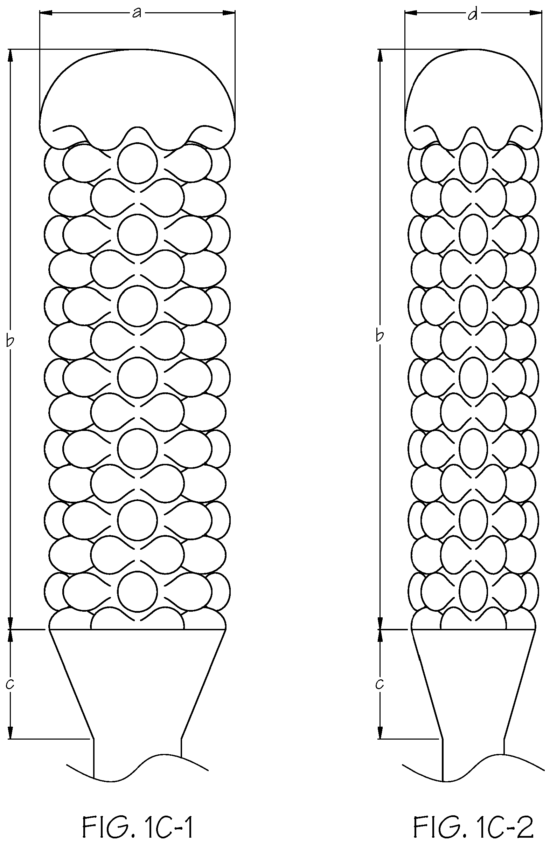

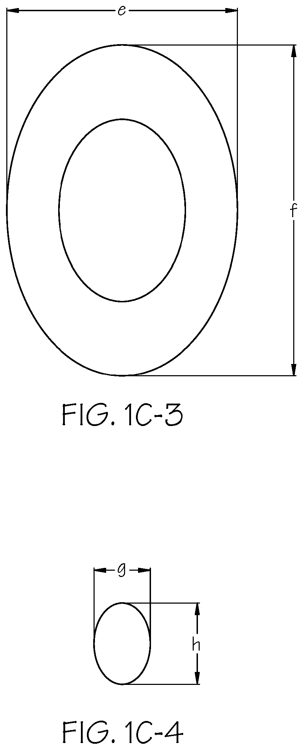

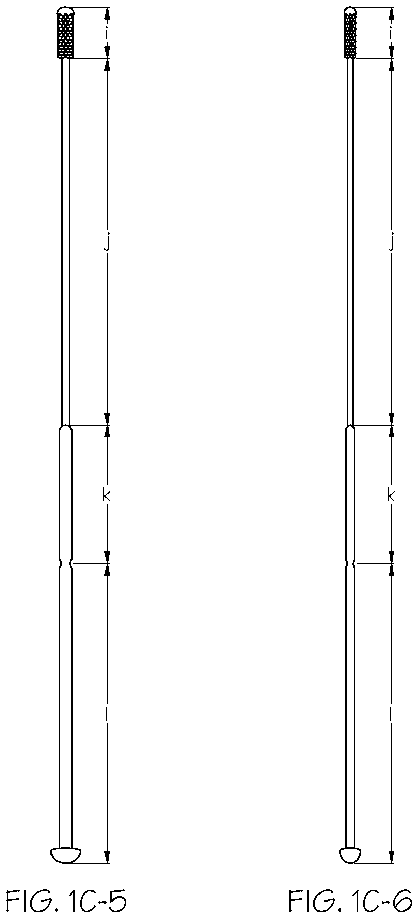

The institutional review board approved all clinical data use. Examination of maxillofacial CT scans (e.g. FIG. 1A) of patients 1-3 years of age suggested that primary deflection of the swab in the sagittal plane would be sufficient to navigate from the external nares to the posterior nasopharynx (e.g. FIG. 1B). Therefore, designed elliptical cross-section swabs (FIG. 1C-4) and verified that sagittal shaft deflection was adequate. 3D prints of the nasopharyngeal passage itself were made, and the ability of individual swab prototypes to navigate the passage was physically tested. It was noted that less flexible designs would get trapped in the crenulations of the nasopharyngeal passage and might require multiple attempts to reach the posterior nasopharynx, thus lengthening the time required to reach the sampling location and possibly injuring the delicate pediatric anatomy. On the assumption that increased navigation time and resistance correlated with patient discomfort, the navigation time and a qualitative assessment of resistance were chosen as measures of patient discomfort, ultimately selecting the design with the shortest navigation time and the lowest resistance score for clinical use. However, other designs were also suitable as swabs for children.

Maxillofacial CT datasets of 5 patients, 11 to 34 months of age and unremarkable for pathologies involving the maxillofacial region, were randomly selected from our data base. Images were processed in 3D Slicer (Version 4.10; http://www.slicer.org) to create Standard Triangle Language files for printing. Nasopharyngeal passages were printed on a Form2 printer using Elastic Resin (Formlabs). Swab designs were made in Fusion 360 (AUTODESK) and printed on a Form2 printer using Surgical Guide Resin (Formlabs).

5 swabs designs were tested: 2 commercial prior art mini-swabs, Flocked 1, PURITAN Diagnostics and Flocked 2, COPAN Flock Technologies; and 3 in-house-printed, all of which had a flexible shaft length of approximately 50 mm, a handle of 78 mm, and a transition zone of 14 mm, as shown in FIGS. 1C-5 & 1C-6, and varied in the flexible shaft cross-section: Design E, 1.2 mm x 0.8 mmø; Design I, 1.2 mm; and Design ES, 1.2 mm x 0.8 mmø, with an additional slanted posterior edge of the brush. Table II below shows dimensions (in millimeters) for each of the swab types tested. Navigation of each swab from the external nares to the posterior nasopharynx was done by 1 individual, the order of testing of the individual swabs and anatomic models was randomized, and each test was repeated 3 times. The time to complete navigation was timed by an observer using a hand-held stopwatch, while the resistance on insertion was classified using a 3-level resistance score (1, easy insertion: no resistance; 2, medium: mild resistance; and 3, hard: resistance requiring extra force). All statistical calculations were performed in Matlab R19a (MathWorks).

**TABLE II: Tabularized dimensions for 2 commercial and 3 in-house printed swab designs**

| Swab | a | b | c | d | e | f | g | h | i | j | k | l |
|---|---|---|---|---|---|---|---|---|---|---|---|---|
| Flocked 1 | 2.3 | 7.0 | 0 | 2.3 | 2.5 | 2.5 | 1.2 | 1.2 | 7.0 | 44 | 8.3 | 94 |
| Flocked 2 | 2.7 | 7.4 | 0 | 2.7 | 2.5 | 2.5 | 0.9 | 0.9 | 7.4 | 66 | 24.5 | 53 |
| Design E | 2.7 | 8.5 | 0 | 1.9 | 3.5 | 5.0 | 0.8 | 1.2 | 8.5 | 50.8 | 14.3 | 78.4 |
| Design I | 2.7 | 8.5 | 0 | 2.6 | 5.0 | 5.0 | 1.2 | 1.2 | 8.5 | 50.8 | 14.3 | 78.4 |
| Design ES | 2.7 | 8.5 | 1.8 | 1.9 | 3.5 | 5.0 | 0.8 | 1.2 | 10.4 | 49 | 14.3 | 78.4 |

| | | | | | | | | | | | | |
|---|---|---|---|---|---|---|---|---|---|---|---|---|
| Dimensions are in millimeters. Columns represents dimensions as shown in FIGS. 1C-1 to 1C-6. | | | | | | | | | | | | |

The quality of specimen recovery from Design ES nasopharyngeal swabs was determined by performing a crossover collection study with the commercial swabs (Puritan Diagnostics) and prototype NB swabs in 2 healthy adult volunteers, a 26-year-old man and a 28-year-old woman. Both nostrils of the volunteers were sampled by each swab in successive order. The swabs were placed into a MicroTest M4RT viral transport containing 3 mL of media (Remel). Nucleic acid was extracted using 200 uL from each viral transport on the eMAG system (bioMérieux) and eluted into 50 uL of buffer. Real-time polymerase chain reaction (RT-PCR) was performed on each eluate using the Centers for Disease Control's 2019- Novel Coronavirus (2019-nCoV) Emergency Use Authorization assay (Centers for Disease Control and Prevention. CDC 2019-Novel Coronavirus (2019-nCoV) Real-Time RT-PCR. U.S. Food and Drug Administration emergency use authorization instructions for use. https://www.fda.gov/media/134922/download. Accessed July 17, 2020), which includes 2 targets in the Severe Acute Respiratory Syndrome coronavirus 2 nucleocapsid region and 1 target in the human RNase P gene to assess the cellular quality of the sample.

### DISCUSSION

This brief report has several limitations. Only 5 nasopharyngeal prints of patients in a limited age range (1-3 years) were used for the testing. A broader range of ages and a larger number of patients would make this study more rigorous. The insertion tests were performed by 1 individual, a technologist with intimate knowledge of the nasopharyngeal anatomy. Testing by a medical professional who would routinely use such swabs in daily practice may change the results. Clinical validation was only performed on 2 adult volunteers and not on a larger number of pediatric subjects. All of these, however, are compromises we made for this emergency development.

### RESULTS

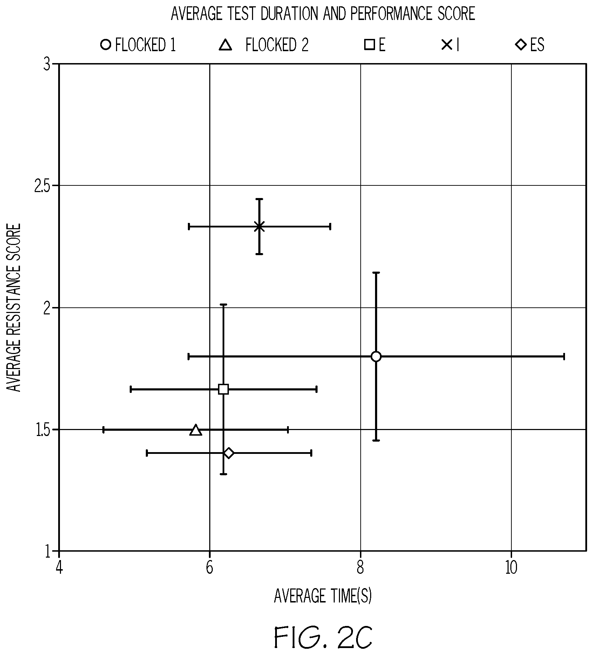

Two commercial flocked mini-swabs (from Puritan Diagnostics and COPAN Flock Technologies) and 3 in-house printed swab designs were tested. FIGS. 2A-2C show average resistance scores, average time, and average test duration and performance scores for the swabs tested. All in-house swabs were successfully navigated to the posterior nasopharynx in all 5 nasopharyngeal passage models, while 1 commercial swab (COPAN Flock Technologies) was incapable of insertion into anatomic model 1 (FIG. 2A). On the basis of the shortest navigation time and the lowest resistance score (FIG. 2C), Design ES was chosen as the preferred design for clinical testing. Preliminary clinical testing in 2 adult volunteers demonstrated recovery of cellular material from Design ES swabs to be within a concentration of 0.3 log of that from the currently used nasopharyngeal swabs.

Embodiments described herein are included to demonstrate particular aspects of the present disclosure. It should be appreciated by those of skill in the art that the embodiments described herein merely represent exemplary embodiments of the disclosure. Those of ordinary skill in the art should, in light of the present disclosure, appreciate that many changes can be made in the specific embodiments described, including various combinations of the different elements, components, steps, features, or the like of the embodiments described, and still obtain a like or similar result without departing from the scope of the present disclosure. From the foregoing description, one of ordinary skill in the art can easily ascertain the essential characteristics of this disclosure, and without departing from the scope thereof, can make various changes and modifications to adapt the disclosure to various usages and conditions. The embodiments described hereinabove are meant to be illustrative only and should not be taken as limiting of the scope of the disclosure.

## Claims

1. A nasopharyngeal swab for pediatric use, the swab comprising:
a tip section (i) that is elliptic-cylindrically shaped;
a shaft section (j) below the tip section that is elliptic-cylindrically shaped, wherein shaft section is flexible for pediatric use;
a handle section (l) below the shaft section, wherein the handle section has larger cross-sectional dimensions that the shaft section; and
a transition section (k) that transitions the shaft section to the handle section,
**characterized in that** the tip section provides an arrangement of hemispherical nubs about a peripheral surface of the tip section.

2. The swab of claim 1, wherein a semi-major axis and a semi-minor axis of the shaft section are equal to or less than 1.2 mm.

3. The swab of claim 2, wherein the semi-minor axis of the shaft section is equal to or less than 0.8 mm.

4. The swab of claim 1, wherein a length of the shaft section is 51 mm or less.

5. The swab of claim 1, wherein a semi-major axis and a semi-minor axis of the tip section are equal to or less than 2.7 mm.

6. The swab of claim 1, wherein a nubbed section (b) of the tip section is 8.5 mm in length.

7. The swab of claim 1, wherein the tip section provides a transition tip section (c) where a size of the tip reduces to a desired size of the shaft section.

8. The swab of claim 7, wherein the transition tip section has a length of 1.8 mm or less.

9. The swab of claim 1, wherein the transition section (k) has a length of 14.3 mm.

10. The swab of claim 1, wherein the handle section has a length of 78.4 mm.

11. The swab of claim 1, wherein the tip section is unflocked.

12. The swab of claim 1, wherein the swab is 3D printed, molded, injection molded, reaction injection molded, machined by subtractive machining methods, or any combination of the preceding.

## Patentansprüche

1. Nasopharyngealer Tupfer zur pädiatrischen Verwendung, wobei der Tupfer Folgendes umfasst:
einen Spitzenabschnitt (i), der elliptisch-zylindrisch geformt ist;
einen Schaftabschnitt (j) unter dem Spitzenabschnitt, der elliptisch-zylindrisch geformt ist, wobei Schaftabschnitt flexibel für pädiatrische Verwendung ist;
einen Griffabschnitt (l) unter dem Schaftabschnitt, wobei der Griffabschnitt größere Querschnittsabmessungen als der Schaftabschnitt aufweist; und
einen Überführungsabschnitt (k), der den Schaftabschnitt in den Griffabschnitt überführt,
**dadurch gekennzeichnet, dass** der Spitzenabschnitt eine Anordnung von halbkugelförmigen Noppen um eine Umfangsfläche des Spitzenabschnittes bereitstellt.

2. Tupfer nach Anspruch 1, wobei eine große Halbachse und eine kleine Halbachse des Schaftabschnittes gleich oder weniger als 1,2 mm sind.

3. Tupfer nach Anspruch 2, wobei die kleine Halbachse des Schaftabschnittes gleich oder weniger als 0,8 mm ist.

4. Tupfer nach Anspruch 1, wobei eine Länge des Schaftabschnittes 51 mm oder weniger ist.

5. Tupfer nach Anspruch 1, wobei eine große Halbachse und eine kleine Halbachse des Spitzenabschnittes gleich oder weniger als 2,7 mm sind.

6. Tupfer nach Anspruch 1, wobei ein genoppter Abschnitt (b) des Spitzenabschnittes 8,5 mm lang ist.

7. Tupfer nach Anspruch 1, wobei der Spitzenabschnitt einen Überführungsspitzenabschnitt (c) bereitstellt, an dem sich eine Größe der Spitze auf eine gewünschte Größe des Schaftabschnittes reduziert.

8. Tupfer nach Anspruch7, wobei der Überführungsspitzenabschnitt eine Länge von 1,8 mm oder weniger aufweist.

9. Tupfer nach Anspruch 1, wobei der Überführungsabschnitt (k) eine Länge von 14,3 mm aufweist.

10. Tupfer nach Anspruch 1, wobei der Griffabschnitt eine Länge von 78,4 mm aufweist.

11. Tupfer nach Anspruch 1, wobei der Spitzenabschnitt unbeflockt ist.

12. Tupfer nach Anspruch 1, wobei der Tupfer 3D-gedruckt, geformt, spritzgegossen, reaktionsspritzgegossen, bearbeitet durch subtraktive Bearbeitungsverfahren oder eine beliebige Kombination des Vorstehenden ist.

## Revendications

1. Écouvillon nasopharyngien destiné à un usage pédiatrique, l'écouvillon comprenant :
une section de pointe (i) de forme elliptique-cylindrique ;
une section de tige (j) sous la section de pointe qui est de forme elliptique-cylindrique, ladite section de tige étant souple destinée à un usage pédiatrique ;
une section de manche (l) en dessous de la section de tige, ladite section de manche comportant des dimensions transversales plus grandes que la section d'arbre ; et
une section de transition (k) qui fait passer la section de tige à la section de manche, **caractérisé en ce que** la section de pointe fournit un agencement de protubérances hémisphériques autour d'une surface périphérique de la section de pointe.

2. Écouvillon de la revendication 1, un demi-grand axe et un demi-petit axe de la section de tige étant inférieurs ou égaux à 1,2 mm.

3. Écouvillon de la revendication 2, ledit demi-petit axe de la section de tige étant inférieur ou égal à 0,8 mm.

4. Écouvillon de la revendication 1, une longueur de la section de tige étant inférieure ou égale à 51 mm.

5. Écouvillon de la revendication 1, un demi-grand axe et un demi-petit axe de la section de tige étant inférieurs ou égaux à 2,7 mm.

6. Écouvillon de la revendication 1, une section à protubérances (b) de la section de pointe possédant une longueur de 8,5 mm.

7. Écouvillon de la revendication 1, ladite section de pointe fournissant une section de pointe de transition (c) où une taille de la pointe se réduit à une taille souhaitée de la section de tige.

8. Écouvillon de la revendication 7, ladite section de pointe de transition comportant une longueur inférieure ou égale à 1,8 mm.

9. Écouvillon de la revendication 1, ladite section de transition (k) comportant une longueur de 14,3 mm.

10. Écouvillon de la revendication 1, ladite section de manche comportant une longueur de 78,4 mm.

11. Écouvillon de la revendication 1, ladite section de pointe étant non floquée.

12. Écouvillon de la revendication 1, ladite écouvillon étant imprimé en 3D, moulé, moulé par injection, moulé par injection et réaction, usiné par des procédés d'usinage soustractif, ou toute combinaison de ce qui précède.
